# EUROPEAN PATENT APPLICATION

(11) **EP 4 000 733 A1**
(43) Date of publication of application: **25.05.2022**
(21) Application number: 21208600.3
(22) Date of filing: 16.11.2021
(51) Int. Cl.: B01L 7/00, B64D 11/04, G01N 1/22

(54) **AIRCRAFT GALLEY INSERT WITH PATHOGENS DIAGNOSTIC TEST CAPABILITIES**

(30) Priority: 16.11.2020 US 202063114366 P; 16.11.2020 US 202063114330 P; 16.11.2020 US 202063114339 P; 16.11.2020 US 202063114350 P; 16.11.2020 US 202063114400 P; 16.11.2020 US 202063114064 P; 16.11.2020 US 202063114157 P; 16.11.2020 US 202063114386 P
(71) Applicant: Koninklijke Fabriek Inventum B.V., 3439 MG Nieuwegein (NL)
(72) Inventor: GONZALEZ, Arnau Castillo, 3607 AK Maarsen (NL); MURCIA, Antonio Martinez, 033317 Orihuela (ES)
(74) Representative: Dehns

(57) **Abstract**

A modular aircraft galley insert for performing thermal cycling for PCR testing includes a housing having a sample module (104) for receiving a sample to be tested, a thermal module (106) thermally connected to the sample module for cycling temperatures of the sample to be tested, a power module (108) for providing power to the thermal module, a detector module (110) configured to analyze the sample and connected to receive power from the power module, and a connectivity module (112) for communicating results of a test from the detector module to a remote location with respect to the housing.

## Description

### Technological Field

The present application is related to a system and method used to collect a representative air sample of an aircraft, more specifically to a method, and systems for analyzing a biological sample on an aircraft through a pathogen/contaminant diagnostic test such as real time PCR test.

### Description of Related Art

The spread progression of SARS-CoV-2 around the world has risen a red flag: Economic economic globalization creates systemic risks. As trade, finance, travel, cyber and other networks grow in scale and interact, they become more complex and unstable. The transporters of the goods of the global economy, such as major airport hubs, are also spreaders of the pathogens. The 2008 global financial crisis provided a dramatic example of how contagions could spread from the US to global markets overnight. So too has the rapid spread of cyber viruses. In health, rising life expectancy and success in preventing a repeat of the devastating influenza pandemic of 1918, which infected about one-third of the world's population and killed as many as 50m people, has created a false sense of security. But the world is now more interdependent. For example, China represents almost one-fifth of global output, is integral to global supply chains, and its tourists spend over $260 billion annually. The COVID-19 pandemic shed light on the need for better monitoring, detecting, and isolating ill passengers, specifically due to the havoc that was wreaked detrimental impact on the global economy, specifically air travel due to closed borders, movement restrictions, and testing requirements.

However, the COVID-19 pandemic the air travel industry has proven that air travel can be safe and that aircraft cabins have a well-managed airflow that inhibits minimize the risk for transmission of virus, and that being seated onboard an aircraft is safer than shopping in large stores. Governments and other authorities need to assume that aircraft are contaminated until proven "clean", as 25% of COVID-19 cases are asymptomatic or pre-symptomatic; but still contagious. Thus, if borders shutdown and there is a drastic reduction in international travel, global passenger travel is greatly reduced. To date travelers and governments have relied on individual diagnostic tests. The uncertainty of the results has reduced people's inclination to travel and subsequent airline inclination to maintain routes.

Accordingly, conventional systems and methods of monitoring infections has not lived up to requirements of the fast-paced modern world. Thus, there is still a need in the art for an improved on-board virus and pathogen detection system. The present disclosure provides a solution for this need.

### Summary of the Invention

A modular aircraft galley insert for performing pathogen/contaminant diagnostic testing such as thermal cycling for PCR testing is disclosed. The insert includes a housing having, a sample module for receiving a sample to be tested, a sample concentration module, a purification and extraction module, a thermal module thermally connected to the sample module for cycling temperatures of the sample to be tested, a power module for providing power to the different modules, a detector module configured to analyze the amplified signal obtained from the sample and connected to receive power from the power module, the signal should be detected after each amplification cycle, and a connectivity module for communicating results of a test from the detector module to a remote location with respect to the housing.

A storage space can be located within the housing for storing consumables and process waste. The waste generated is not medical waste. The consumables are inert, water based, and safe for the environmental. The samples do not contain human biological information (DNA/RNA). A second storage space can be present for storing the tested sample. A module may be provided for sample automatic loading. This module is configured to transfer a portion of the purified sample through a micro-pipette tip propelled by a pump and this aliquot deposited over each vessel to run a PCR test.

The power rating of the insert is about 1000W. A volume of the insert is sized to fit within a galley ARINC size between 4 and 6. The insert can operate independently of the cabin pressure ranges or other environmental conditions in the aircraft. The insert can be inserted within a galley monument for a cabin area of an aircraft, where the monument includes a first monument stack, a second monument stack adjacent to the first monument stack, and a tray within the second monument stack to secure the insert.

The connectivity module can be connected to a cockpit of an aircraft. The power module can be connected to an aircraft power source. The insert can be integrated within a monument stack, or the insert can be removably attached to a monument stack.

These and other features of the systems and methods of the subject disclosure will become more readily apparent to those skilled in the art from the following detailed description of the preferred embodiments taken in conjunction with the drawings.

### Brief Description of the Drawings

So that those skilled in the art to which the subject disclosure appertains will readily understand how to make and use the devices and methods of the subject disclosure without undue experimentation, embodiments thereof will be described in detail herein below with reference to certain figures, wherein:
- FIG. 1: is an isometric view of an insert for PCR testing according to the disclosure; and
- FIG. 2: is a schematic view of the modules of the insert.

### Detailed Description

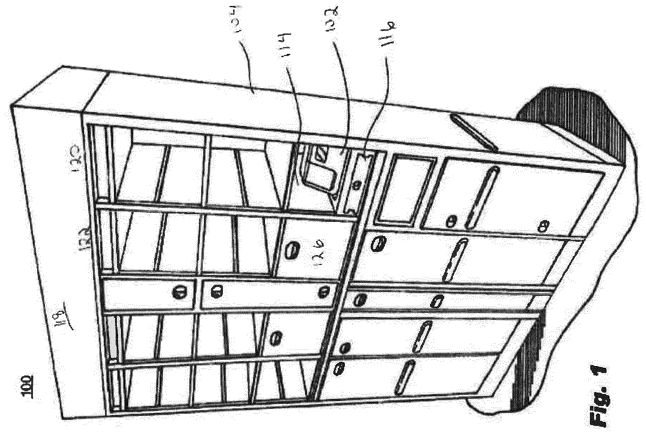

Reference will now be made to the drawings wherein like reference numerals identify similar structural features or aspects of the subject disclosure. For purposes of explanation and illustration, and not limitation, a partial view of an exemplary embodiment of a system in accordance with the disclosure is shown in FIG. 1 and is designated generally by reference character 100. The system described below is used to test a bulk sample, representative of all passengers on the aircraft and to test it to provide a bulk screening of the aircraft prior to arrival of the aircraft at a destination.

FIG. 1 shows a modular aircraft galley insert 100 for performing thermal cycling for PCR testing. A storage space 114 is located within the housing 104 for storing consumables and waste. The waste generated is not medical waste. The consumables are inert, water based, and safe for the environmental. A second storage space 116 can be present for storing the tested sample after a test is complete. The insert 102 is inserted within a galley monument 118. The monument 118 includes a first monument stack 120, a second monument 122 stack adjacent to the first monument stack 120, and a tray 126 within the second monument stack 124 to secure the insert 102.

The power rating of the insert 100 is about 1000W. A volume of the insert is sized to fit within a galley ARINC size between 4 and 6. The insert can operate independently of the cabin pressure ranges or other environmental conditions in the aircraft.

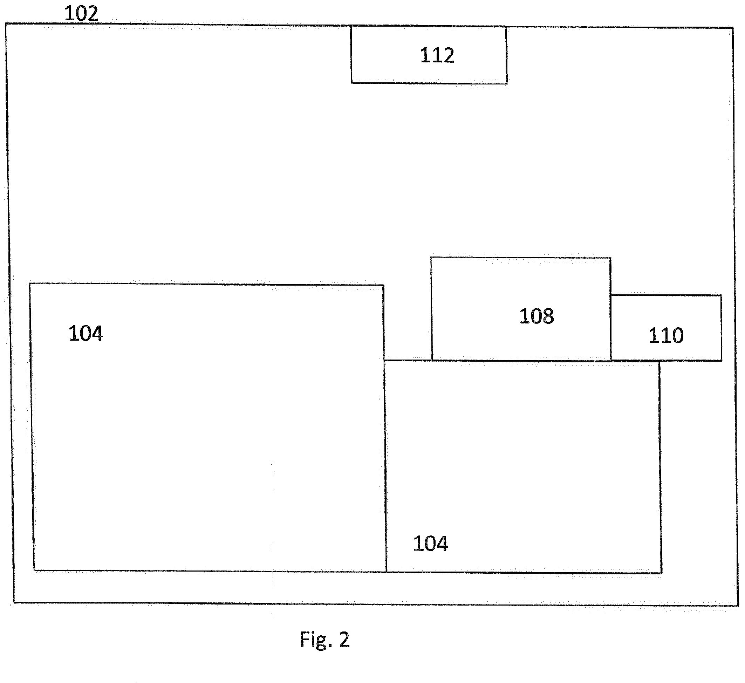

Referring to FIG. 2, the insert 100 includes a housing 102 that includes a sample module 104 for receiving a sample to be tested, a thermal module 106 thermally connected to the sample module 104 for cycling temperatures of the sample to be tested, a power module 108 for providing power to the thermal module, a detector module 110 configured to analyze the sample and connected to receive power from the power module 108, and a connectivity module 112 for communicating results of a test from the detector module 110 to a remote location with respect to the housing 102. The connectivity module 112 can be connected to a cockpit of an aircraft in order to relay the results of the test to the crew and pilots. It is also considered that the connectivity module is connected to communicate to a destination or origin airport to relay the results of the test. The power module 108 can be connected to an aircraft power source.

The reagents that are present within the insert 102, being ready to use, are mixed, and stable at room temperatures. Reagents will be pathogen-specific, that is, a determined set of reagents for each specific pathogen to be detected. Protocols for running programs are programed in the unit for PCR cycling: a few examples are the setup Temperatures of the specific thermal cycle, timing or number of cycles; in the case of the thermal module.

The methods and systems of the present disclosure, as described above and shown in the drawings, provide for an improved bulk data and analysis of passenger pathogens on an aircraft. The system provides a more efficient primary health control at airports, helps identify at early stages emerging pathogens and origin of outbreaks, and empower their scientific research (either academic and/or commercial / institutional). While the apparatus and methods of the subject disclosure have been shown and described with reference to preferred embodiments, those skilled in the art will readily appreciate that changes and/or modifications may be made thereto without departing from the scope of the invention as defined by the claims.

## Claims

1. A modular aircraft galley insert for performing thermal cycling for PCR testing comprising:
a housing (102) having:
a sample module (104) for receiving a sample to be tested;
a thermal module (106) thermally connected to the sample module for cycling temperatures of the sample to be tested;
a power module (108) for providing power to the thermal module;
a detector module (110) configured to analyze the sample and connected to receive power from the power module; and
a connectivity module (112) for communicating results of a test from the detector module to a remote location with respect to the housing.

2. The insert of claim 1, further comprising a first storage space (114) for storing consumables and process waste materials within the housing.

3. The inset of claim 1 or 2, further comprising a second storage space (116) for storing the tested sample.

4. The insert of any preceding claim, further comprising a loading module for sample automatic loading and configured to transfer a portion of a test sample through a pipette tip for testing purposes.

5. The insert of claim 4, wherein the loading module further comprises a pump.

6. The insert of claim 4, wherein the loading module further comprises a pipette.

7. The inset of any preceding claim, wherein the power rating of the insert is approximately 1000W.

8. The insert of any preceding claim, wherein further comprising a plurality of testing vessels configured to receive an individual sample within the housing.

9. The insert of any preceding claim, wherein the insert is configured to operate independently of the cabin pressure ranges or other environmental conditions in the aircraft.

10. The insert of any preceding claim, wherein the insert is inserted within a galley monument (118) for a cabin area of an aircraft, where the monument includes:
a first monument stack (120);
a second monument stack (124) adjacent to the first monument stack; and
a tray (126) within the second monument stack to secure the insert.

11. The insert of any preceding claim, wherein the connectivity module (112) is connected to a cockpit of an aircraft.

12. The insert of any preceding claim, wherein the connectivity module (112) is connected to an on on-ground- receiver.

13. The inset of any preceding claim, wherein the power module (108) is connected to an aircraft power source.

14. The insert of any preceding claim, wherein the insert (100) is integrated within a monument stack.

15. The insert of any of claims 1 to 13, wherein the insert (100) is removably attached to a monument stack.
